# EUROPEAN PATENT SPECIFICATION

(11) **EP 3 228 273 B1**
(45) Date of publication and mention of the grant of the patent: **25.12.2019**
(21) Application number: 16382149.9
(22) Date of filing: 06.04.2016
(51) Int. Cl.: A61B 90/00, A61M 37/00, B43K 23/08, B43K 27/02, B43K 29/08

(54) **SURGICAL MARKER**
CHIRURGISCHER MARKER
MARQUEUR CHIRURGICAL

(43) Date of publication of application: 11.10.2017
(73) Proprietor: Clinica de Ortodoncia y Cirugia Maxilofacial, S.L., 38003 Santa Cruz de Tenerife (ES)
(72) Inventor: Martínez Gimeno, Carlos, E-38003 Santa Cruz de Tenerife (ES)
(74) Representative: Álvarez López, Sonia

(56) References cited:
- WO-A2-2014/179698
- US-A1- 2007 261 174
- Aspen Surgical: "Marker Information", , 31 December 2013 (2013-12-31), XP055298351, Retrieved from the Internet: URL:https://www.aspensurgical.com/files/re sources/copy_of_permanent__skin_marker_inf ormation_brochur.pdf [retrieved on 2016-08-29]
- Aspen Surgical: "WriteSite Dual Tip Skin Marker", , 28 April 2015 (2015-04-28), pages 1-1, XP055298334, Retrieved from the Internet: URL:https://www.aspensurgical.com/files/re sources/copy_of_writesite_dual_tip_skin_ma rker_-_product_b.pdf [retrieved on 2016-08-29]
- Meredith Melnick: "IKEA Pencils: The Latest in Surgical Technology", TIME, 10 December 2010 (2010-12-10), pages 1-1, XP055298435, Retrieved from the Internet: URL:http://healthland.time.com/2010/12/10/ ikea-pencils-the-latest-in-surgical-techno logy/ [retrieved on 2016-08-30]

## Description

### OBJECT OF THE INVENTION

The present invention relates to a surgical marker. The corresponding field is that of medical-surgical instruments.

### BACKGROUND OF THE INVENTION

Current surgical markers basically consist of sterile pens with different tip thickness and colours, using different ink types (methylene blue, gentian violet, etc.) which mainly work on dry surfaces and which fade or disappear with surgical washing. None of them can paint or mark moist surfaces or bone in a lasting manner.

In order to mark beneath the epidermis markers are known such as that described in US 4,789,582, which comprises a needle cartridge for a surgical pen or tattooing tool with a reservoir filled with a liquid pigment adapted to be discharged to the skin through a needle with three simultaneously acting tips. However, it requires a specific air-driven pen.

Document US5496304 discloses a device and method comprising a member for penetrating the outermost layer of the epidermis and a reservoir supplying a marking agent, such that as the penetrating member enters the epidermis the marking agent is introduced under the outermost layer thereof, using as a marker, for example, methylene blue. The penetrating member is shaped as a wheel with tips to mark lines. It is therefore not suitable for marking points or marks that do not require lengthy displacements of the marker, such as in anatomical parts with folds or reduced space, and can only be used on the skin. A similar construction and advantages and drawbacks are present in the marker of US5713890 and that of US2004/0116907.

Document US5909978 describes a marker comprising a cartridge with inks, airtight except at an opening in the distal end -to receive the tip of a marker- and a ventilation orifice, wherein the walls of the pen included in the marker are flexible in order to eject the ink, which is also regulated by partially covering the ventilation orifice. The purpose of this is to force the exit and generate a constant flow of ink, preventing the drawback of tip obstruction or failure of operation because the tip is mixed with grease or tissue remains; however, this marker cannot inject subcutaneously.

Also known is US6197034, which describes a marker that can be used on soft and hard mammalian tissue, as well as a pen containing a medical marking device and methods for using said pens to make lines and marks on soft and hard mammalian tissue. The pen can be used for other surgical marking applications and is used as a normal writing implement. It comprises a housing with an inner reservoir containing inks and two ends, one of which is closed while the opposite end has an extended member with a groove, and a hollow tube coaxial with the groove. Inside the reservoir is a vertically movable mass joined to a stem with a length somewhat greater than that of the hollow tube, such that the mass blocks the opening of the inks unless the stem is pushed from above. This is identical to certain old linear drawing devices and has the same drawbacks of the ink drying in the hollow tube, as well as uncontrolled ink outlet in certain situations.

Document US2013/0197358 describes another surgical marker comprising: a pen body with a first chamber containing a powder colouring, a second chamber separated from the first chamber containing a solvent, where the first and second chamber are selectively placed with fluid communication with each other, and a tip in fluid communication with the first or second chamber, wherein at least one powder colouring and the solvent, when mixed, form a colouring solution that can be discarded on the surface of the skin or tissue and can be viewed simultaneously by a user without assistance and with a fluorescent image generator. It can be used to mark areas which previous required fluorescent light generators to be seen, but maintains the specific construction with the various chambers, and does not ensure permanence of the marking with surgical washing.

Also known is document US2015/0119866, which describes a surgical marker for laparoscopic interventions that is connected to a rod and can be pushed through a laparoscopy cannula to the target tissue to mark. The marker can adjust (reduce) its length by moving the location of the ink reservoir from its position in a typical marker to a location on a cover, and comprises a connector with a cap formed therein to receive the pin of a rod and a tip to contain and distribute the ink on the tissue.

Document US 5665092 describes a surgical marker for bones comprising a tubular guide cannula, a tube that can be inserted in the cannula with a proximal end and a distal end, a flexible and movable rod that extends along and inside the tube, and a marking tip connected to a proximal end of the flexible rod and extending beyond the proximal end of the tube, which allows marking the operation site accurately with minimal pain to the patient. Marking is produced by bone erosion and the marker overall has a complex construction and handling.

All of these surgical markers have a specific use, either for subcutaneous marking, linear or otherwise, bone erosion or simple marking, such that their versatility is very limited. In addition, the permanence of the marking with surgical washing is ensured in only a few of them.

US 2007/261174 describes a marker having exchangeable tips according to the preamble of claim 1.

### DESCRIPTION OF THE INVENTION

The invention is defined by the appended independent claim. Preferred embodiments of the invention are illustrated in the dependent claims.

The surgical marker of the invention has a configuration with small multi-surface tips that are exchangeable, allowing to use only those needed according to the technique and tissue to mark, and allowing surgical field markings that are not possible with current devices.

In addition, the marking performed in each case is resistant to surgical washing and provides a lasting marking.

In addition, the marking performed in each case lasts during the work, as the tip of porous material soaked in ink is used with moisture-resistant ink, the graphite tip allows marking on bone even when wet and allows using saws with irrigation without fading and requiring remarking, and in subcutaneous injection the skin itself protects the ink.

### BRIEF DESCRIPTION OF THE DRAWINGS

Figure 1 shows a side view of the marker of the invention with the various tips thereof, where the covers of said tips are sectioned for purposes of clarity.

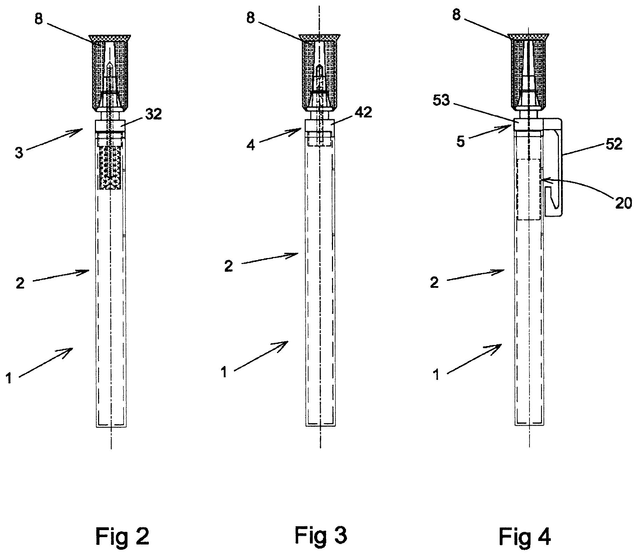

Figures 2, 3 and 4 respectively show a side view of the marker of the invention with the tip of porous material soakable in ink, the graphite tip and the injection tip mounted, marking the concealed lines and with the covers of the tips sectioned.
Figure 5 shows a side, elevation and bottom plan view of the marker of the invention with the injection tip mounted, showing by concealed lines in the elevation view the male protrusion of the exchangeable tip attachment means to the handle inserted in the female notch.
Figure 6 shows an enlarged view of the clipping of one of the tips to the handle of the marker of the invention. This clipping is the same for all the tips.

### DESCRIPTION OF AN EMBODIMENT

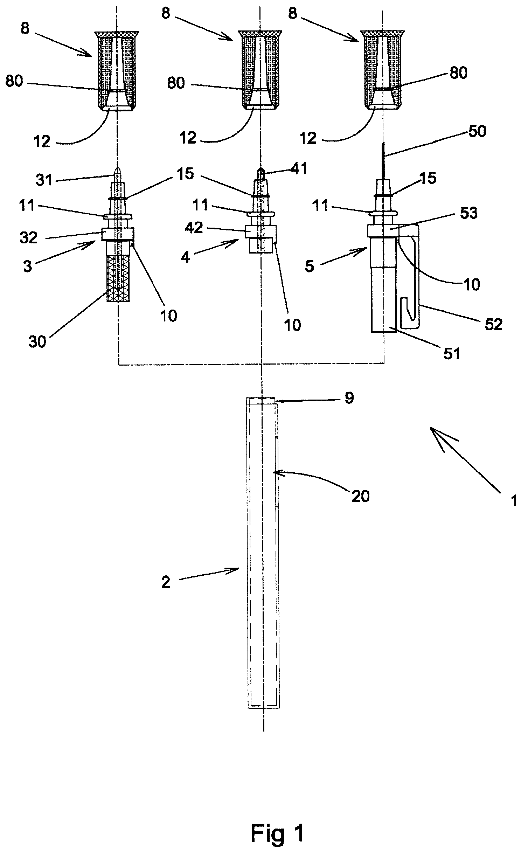

The surgical marker (1) shown in Figure 1 comprises:
- a handle (2) in the form of a marker body, tubular, that can be held in the hand, and
- exchangeable attachment means for exchangeable tips (3, 4, 5) arranged on said handle (2) and at the tips (3, 4, 5),
where the exchangeable tips comprise:
- a tip (3) made of a porous material soakable in ink for marking skin,
- a graphite tip (4) for marking bone, even if it is wet or has blood,
- an injection tip (5) for precision marking of points to design skin plasties by injection in the dermis that do not fade with surgical washing. This tip is particularly useful to mark skin flaps with precise geometry, such as in cleft lips, other plasties with precise geometry and to perform otoplasties.

All of the above such that in a single surgical intervention several tips can be used depending on the technique to perform.

The tips (3) made of a porous material soakable in ink comprise an applicator (31) for said material and a reservoir (30) with the same or similar material, increasing the amount of ink contained and therefore the duration thereof. The applicator (31) and the reservoir (30) are mounted on a first support (32) where the exchangeable attachment means to the handle (2) are implemented. In addition, said tips (3) of a porous material soakable in ink are soaked in moisture-resistant ink and comprise inks of several colours and/or thickness, including at least three different thicknesses: fine, medium and thick.

The graphite tip (4) instead comprises a graphite portion (41) with at least HD hardness, such as to allow an effective bone marking, and is mounted on a second support (42) where the exchangeable attachment means to the handle (2) are implemented.

With regard to the injection tip (5), ideally it comprises a hollow needle (50) for subcutaneous injection of the ink, in communication with an elastic reservoir (51) that can be compressed through an orifice (20) provided on the handle (2) that allows pressing said elastic reservoir (51) applying sufficient pressure to inject the ink subcutaneously through the hollow needle (51). Said hollow needle (50) and the reservoir (51) are mounted on a third support (53) where the exchangeable attachment means to the handle (2) are implemented. In addition, said third support (53) preferably comprises a flexible elastic lever (52) that can recover its position coinciding with the orifice (20) and with the reservoir (51) in order to compress the latter more easily, the orifice (20) located on the end of the handle (2) where the exchangeable attachment means of the tips (3, 4, 5) are provided, and at the same position as the reservoir (51), such that a reservoir with sufficient volume is obtained, yet not too big for the ink to dry or the pressure exerted to be dispersed. The ink used in the injection point (5) can be methylene blue or gentian violet, which will not fade with surgical washing, remaining at the initially intended position even if the wound is already open.

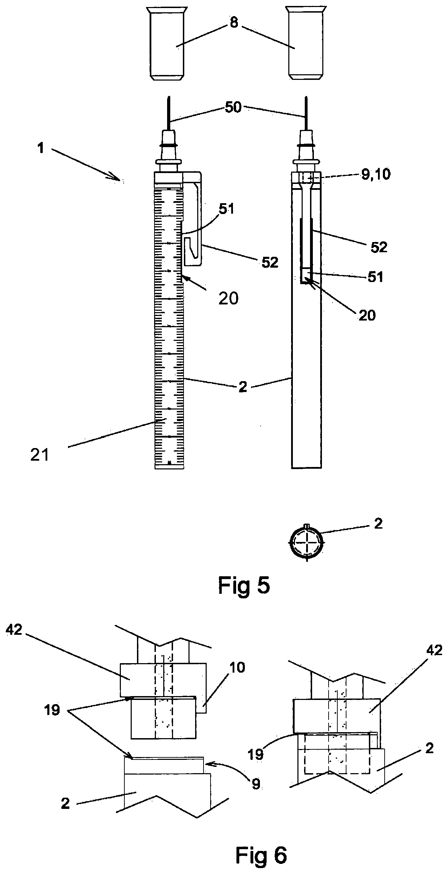

The exchangeable attachment means of the exchangeable tips (3, 4, 5) provided on the handle (2) comprise in this preferred embodiment (see figure 6) a removable annular clip (19) as well as a female notch (9) and a complementary male protrusion (10), such that the union is not rotary and connection is simple and effective Said female notch (9) is provided on the handle (2) and the male protrusion (10) is provided on the tips (3, 4, 5) as shown in the figures, or vice versa.

In addition the handle (2) comprises a ruler (21) (see figure 5) to measure the markings, which can be used with the tips (3, 4, 5) mounted or not mounted on the marker (1). Said ruler (21) ideally has millimetre gradings and can be implemented in the handle (2) by printing and/or moulding.

The tips (3, 4, 5) ideally comprise a lid (8) to prevent the porous material from drying and/or to protect the graphite and the injection needle. Said lid (8) is attached by fitting means to the corresponding tip (3, 4, 5), the fitting means thereof have a smaller tensile resistance than the exchangeable attachment means of the tips (3, 4, 5) (than the annular clip (19) in this example), in order to take out the lid (8) without removing the tip mounted on the handle (2). Said fitting means comprise for example an annular protrusion (11) and an annular fitting (12) for said annular protrusion (11), said annular protrusion (11) being disposed on the tip (3, 4, 5) and the annular fitting in the lid (8), or vice versa.

In addition, the tip (3, 4, 5) comprises a frustoconical sealing lip (15) arranged against an inner recess (80) of the lid (8) to prevent air from entering and drying the ink.

Lastly, the handle (2) and/or the tips (3, 4, 5) are made of plastic to reduce costs, and the tips (3, 4, 5) comprise a preliminary sterilisation treatment and a sterile protection packaging, not shown, for immediate use.

Having sufficiently described the nature of the invention and the embodiment thereof, it should be noted that the arrangements described above and shown in the accompanying drawings are subject to changes in the details thereof provided the fundamental design is not affected.

## Claims

1. Surgical marker (1) comprising:
- an elongated handle (2) for holding with the hand as a writing instrument, and
- attachment means for exchangeable tips (3, 4, 5), provided on said handle and on the tips (3, 4, 5),
where the exchangeable tips comprise:
- a tip (3) made of a porous material soakable in ink for marking skin,
- a graphite tip (4) for marking bone, and
- an injection tip (5) for precision marking of points to design skin plasties by injection in the dermis,
**characterized in that** the injection tip (5) comprises a hollow needle (50) for subcutaneous injection of the ink in communication with an elastic reservoir (51) that can be compressed through an orifice (20) provided on the handle (2); the hollow needle (50) and the reservoir (51) mounted on a third support (53) in which are implemented the attachment means to the handle (2),
where the third support (53) comprises a flexible elastic lever (52) that can restore its position in coincidence with the orifice (20) to compress the reservoir.

2. Surgical marker (1) according to claim 1, where the tips (3) made of a porous material soakable in ink comprise an applicator (31) for said material and a reservoir (30); the applicator (31) and the reservoir (30) mounted on a first support (32) in which are implemented the attachment means to the handle (2).

3. Surgical marker (1) according to any of claims 1 to 2, comprising tips (3) made of a porous material soakable in ink, the tips (3) having different thicknesses.

4. Surgical marker (1) according to claim 1, where the graphite tip (4) comprises a portion of graphite (41) with at least HB hardness, mounted on a second support (42) in which are implemented the attachment means to the handle (2).

5. Surgical marker (1) according to claim 1, where the ink used in the injection point (5) is selected among: methylene blue, or gentian violet.

6. Surgical marker (1) according to any of the preceding claims, where the attachment means provided on the handle (2) comprise a removable annular clip (19) and a female notch (9) and a complementary anti-rotation male protrusion (10); where the female notch (9) is provided on the handle (2) and the male protrusion (10) on the tips (3, 4, 5) or vice versa.

7. Surgical marker (1) according to any of the preceding claims, where the handle (2) comprises a ruler (21) for measuring the markings.

8. Surgical marker (1) according to any of the preceding claims, where the tips (3, 4, 5) comprise a lid (8).

9. Surgical marker (1) according to claim 8, where the lid (8) is attached by fitting means to the corresponding tip (3, 4, 5), the attachment means of which have a lower tensile resistance than the attachment means of the tips (3, 4, 5).

10. Surgical marker (1) according to any of claims 8-9, where the tip (3, 4, 5) comprises a sealing lip (15) placed against an inner recess (80) of the lid (8).

11. Surgical marker (1) according to any of the preceding claims, where the handle (2) and/or the tips (3, 4, 5) are made of plastic materials.

12. Surgical marker (1) according to any of the preceding claims, where the tips (3, 4, 5) comprise a preliminary sterilisation treatment and a sterile protection packaging.

## Patentansprüche

1. Chirurgischer Marker (1), umfassend:
- einen länglichen Griff (2) zum Halten mit der Hand als Schreibwerkzeug und
- Befestigungsmittel für austauschbare Spitzen (3, 4, 5), vorgesehen auf dem Griff und auf den Spitzen (3, 4, 5),
wobei die austauschbaren Spitzen umfassen:
- eine Spitze (3) aus einem porösen Material, die zum Markieren von Haut in Tinte eintauchbar ist,
- eine Graphitspitze (4) zum Markieren von Knochen und
- eine Injektionsspitze (5) zum präzisen Markieren von Punkten, um Hautplastiken zu gestalten, durch Injektion in die Dermis,
**dadurch gekennzeichnet, dass** die Injektionsspitze (5) eine Hohlnadel (50) zur subkutanen Injektion der Tinte in Verbindung mit einem elastischen Speicherbehälter (51) umfasst, der durch eine auf dem Griff (2) vorgesehene Öffnung (20) zusammengedrückt werden kann; wobei die Hohlnadel (50) und der Speicherbehälter (51) auf einem dritten Träger (53) montiert sind, in dem die Befestigungsmittel für den Griff (2) implementiert sind,
wobei der dritte Träger (53) einen flexiblen elastischen Hebel (52) umfasst, der seine Position in Fügung mit der Öffnung (20) wiederherstellen kann, um den Speicherbehälter zusammenzudrücken.

2. Chirurgischer Marker (1) nach Anspruch 1, wobei die Spitzen (3) aus einem porösen Material, die in Tinte eintauchbar sind, einen Applikator (31) für das Material und einen Speicherbehälter (30) umfassen; wobei der Applikator (31) und der Speicherbehälter (30) auf einem ersten Träger (32) montiert sind, in dem die Befestigungsmittel für den Griff (2) implementiert sind.

3. Chirurgischer Marker (1) nach einem der Ansprüche 1 und 2, umfassend Spitzen (3) aus einem porösen Material, die in Tinte eintauchbar sind, wobei die Spitzen (3) unterschiedliche Dicken aufweisen.

4. Chirurgischer Marker (1) nach Anspruch 1, wobei die Graphitspitze (4) einen Graphitteil (41) mit mindestens der Härte HB umfasst, der auf einem zweiten Träger (42) montiert ist, in dem die Befestigungsmittel für den Griff (2) implementiert sind.

5. Chirurgischer Marker (1) nach Anspruch 1, wobei die in dem Injektionspunkt (5) verwendete Tinte aus Folgendem ausgewählt ist: Methylenblau und Enzianviolett.

6. Chirurgischer Marker (1) nach einem der vorhergehenden Ansprüche, wobei die auf dem Griff (2) vorgesehenen Befestigungsmittel eine entfernbare ringförmige Klammer (19) und eine weibliche Kerbe (9) sowie eine komplementäre drehfeste männliche Vorwölbung (10) umfassen; wobei die weibliche Kerbe (9) auf dem Griff (2) und die männliche Vorwölbung (10) auf den Spitzen (3, 4, 5) vorgesehen ist oder umgekehrt.

7. Chirurgischer Marker (1) nach einem der vorhergehenden Ansprüche, wobei der Griff (2) ein Lineal (21) zum Abmessen der Markierungen umfasst.

8. Chirurgischer Marker (1) nach einem der vorhergehenden Ansprüche, wobei die Spitzen (3, 4, 5) einen Deckel (8) umfassen.

9. Chirurgischer Marker (1) nach Anspruch 8, wobei der Deckel (8) an der entsprechenden Spitze (3, 4, 5) durch Passmittel befestigt wird, deren Befestigungsmittel einen geringeren Zugwiderstand aufweisen als die Befestigungsmittel der Spitzen (3, 4, 5).

10. Chirurgischer Marker (1) nach einem der Ansprüche 8 und 9, wobei die Spitze (3, 4, 5) eine Dichtlippe (15) umfasst, die an eine innere Einbuchtung (80) des Deckels (8) angelegt ist.

11. Chirurgischer Marker (1) nach einem der vorhergehenden Ansprüche, wobei der Griff (2) und/oder die Spitzen (3, 4, 5) aus Kunststoffmaterialien bestehen.

12. Chirurgischer Marker (1) nach einem der vorhergehenden Ansprüche, wobei die Spitzen (3, 4, 5) eine Vorsterilisierungsbehandlung und eine Sterilschutzverpackung umfassen.

## Revendications

1. Marqueur chirurgical (1) comprenant :
- une poignée allongée (2) pour tenir avec la main comme un instrument d'écriture, et
- des moyens de fixation pour pointes interchangeables (3, 4, 5), fournis sur ladite poignée et sur les pointes (3, 4, 5),
où les pointes échangeables comprennent :
- une pointe (3) fabriquée d'un matériau poreux imprégnable dans l'encre pour marquer la peau,
- une pointe en graphite (4) pour marquer l'os, et
- une pointe d'injection (5) pour le marquage de précision des points pour concevoir des plasties cutanées par injection dans le derme,
caractérisé parce que la pointe d'injection (5) comprend une aiguille creuse (50) pour injection sous-cutanée de l'encre en communication avec un réservoir élastique (51) qui peut être comprimé à travers d'un orifice (20) fourni sur la poignée (2) ; l'aiguille creuse (50) et le réservoir (51) montés sur un troisième support (53) dans lequel sont appliqués les moyens de fixation à la poignée (2),
où le troisième support (53) comprend une manette élastique flexible (52) qui peut rétablir sa position en coïncidence avec l'orifice (20) pour comprimer le réservoir.

2. Marqueur chirurgical (1) selon la revendication 1, où les pointes (3) en un matériau poreux imprégnable dans l'encre comprennent un applicateur (31) pour ledit matériau et un réservoir (30) ; l'applicateur (31) et le réservoir (30) montés sur un premier support (32) dans lequel sont appliqués les moyens de fixation à la poignée (2).

3. Marqueur chirurgical (1) selon l'une des revendications 1 à 2, comprenant des pointes (3) en un matériau poreux imprégnable dans l'encre, les pointes (3) ayant différentes épaisseurs.

4. Marqueur chirurgical (1) selon la revendication 1, où la pointe en graphite (4) comprend une partie en graphite (41) ayant au moins une dureté HB, montée sur un deuxième support (42) dans lequel sont appliqués les moyens de fixation sur la poignée (2).

5. Marqueur chirurgical (1) selon la revendication 1, où l'encre utilisée au point d'injection (5) est choisie parmi : le bleu de méthylène ou le violet de gentiane.

6. Marqueur chirurgical (1) selon l'une des revendications précédentes, où les moyens de fixation fournis sur la poignée (2) comprennent un clip annulaire amovible (19) et une entaille femelle (9) et une saillie mâle complémentaire anti-rotation (10) ; où l'entaille femelle (9) est fournie sur la poignée (2) et la saillie mâle (10) sur les pointes (3, 4, 5) ou vice versa.

7. Marqueur chirurgical (1) selon l'une des revendications précédentes, où la poignée (2) comprend une règle (21) pour mesurer les marquages.

8. Marqueur chirurgical (1) selon l'une des revendications précédentes, où les pointes (3, 4, 5) comprennent un couvercle (8).

9. Marqueur chirurgical (1) selon la revendication 8, où le couvercle (8) est fixé par des moyens appropriés à la pointe correspondante (3, 4, 5), dont les moyens de fixation présentent une résistance à la traction inférieure à celle des moyens de fixation des pointes (3, 4, 5).

10. Marqueur chirurgical (1) selon l'une des revendications 8-9, où la pointe (3, 4, 5) comprend une lèvre d'étanchéité (15) placée contre un renforcement intérieur (80) du couvercle (8).

11. Marqueur chirurgical (1) selon l'une des revendications précédentes, où la poignée (2) et/ou les pointes (3, 4, 5) sont fabriquées en plastique.

12. Marqueur chirurgical (1) selon l'une des revendications précédentes, où les pointes (3, 4, 5) comprennent un traitement de stérilisation préalable et un emballage de protection stérile.
